# EUROPEAN PATENT APPLICATION

(11) **EP 2 055 303 A2**
(43) Date of publication of application: **06.05.2009**
(21) Application number: 09002742.6
(22) Date of filing: 08.07.1999
(51) Int. Cl.: A61K 31/445, A61K 9/20

(54) **8-chloro-6, 11-dihydro-11-(4-piperidylidene)-5H-benzo [5,6] cyclohepta [1,2-b] pyridine oral compositions**

(30) Priority: 10.07.1998 US 113232
(62) Divisional of application: 03023240.9
(71) Applicant: Schering Corporation, Kenilworth, NJ 07033-0530 (US)
(72) Inventor: Kou, Jim H., Basking Ridge, NJ 07920 (US)
(74) Representative: HOFFMANN EITLE

(57) **Abstract**

Stable pharmaceutical compositions containing 8-chloro-6,11-dihydro-11-(4-piperidylidene)-5H-benzo[5,6]cyclohepta[1,2-b]pyridine ("DCL") and a DCL protective amount of a pharmaceutically acceptable basic salt such as calcium dibasic phosphate and an amount of at least one disintegrant, preferably two disintegrants such as microcrystalline cellulose and starch sufficient to provide dissolution of at least about 80% by weight of the pharmaceutical composition in about 45 minutes and suitable for oral administration to treat allergic redactions in mammals such as man are disclosed.

## Description

### Background of the Invention

This invention relates to pharmaceutical compositions containing 8-chloro-6,11-dihydro-11-(4-pipehdylidene)-5H-benzo[5,6]-cyclohepta[1,2-b]pyridine (hereinafter "descarbonylethoxyloratadine" or ("DCL") and substantially free of DCL decomposition products, and suitable for oral administration to treat allergic reactions in mammals.

U.S. Patent No. 4,659,716 discloses descarbonylethoxy-loratadine which possesses antihistaminic properties with substantially no sedative properties. This U.S. Patent also discloses methods of making descarbonyl-ethoxyloratadine; pharmaceutical compositions it and methods of using the compositions to treat allergic reactions in mammals.

U.S. Patent No 5,595,997 discloses pharmaceutical compositions and methods for treating allergic rhinitis using descarbonylethoxyloratadine. Co-pending, commonly-owned U.S. Patent application Serial No. 08/886,766, filed 07/02/97 discloses polymorphs of descarbonyl-ethoxyloratadine and pharmaceutical compositions containing them.

We are aware of no prior art that discloses the pharmaceutical compositions of the present invention.

There is a need to produce pharmaceutical compositions suitable for oral administration to mammals and containing descarbonylethoxy-loratadine having constant chemical and physical properties in accordance with exacting health registration requirements of the U.S. and international health registration authorities, e.g. , the FDA's Good Manufacturing Practices ("GMP"). requirements and the International Conference on Harmonization ("ICH") Guidelines.

### Summary of the Invention

We have found that descarbonylethoxyloratadine discolors and decomposes in the presence of excipients disclosed in the prior art. We have discovered that these problems are substantially solved when the use of an acidic excipient is avoided and descarbonylethoxyloratadine is combined with a pharmaceutically acceptable carrier medium comprising a DCL-protective amount of a pharmaceutically acceptable basic salt. Thus, this invention provides a pharmaceutical composition comprising an anti-allergic effective amount of descarbonylethoxyloratadine in a pharmaceutically acceptable carrier medium comprising a DCL-protective amount of a pharmaceutically acceptable basic salt.

The pharmaceutical compositions of the present invention contain less than about 1 % of decomposition products such as N-formylDCL initially, as well as when such compositions are stored at 25°C and about 60 % relative humidity for period of at least 24 months.

In a preferred embodiment, this invention provides a pharmaceutical composition comprising an anti-allergic effective amount of descarbonylethoxy-loratadine in a pharmaceutically acceptable carrier medium wherein said composition contains less than about 1% by weight of N-formyl DCL, preferably less than about 0.8% of N-formyl DCL, and more preferably less than about 0.6% of N-formyl DCL.

In another preferred embodiment, this invention provides a pharmaceutical composition for oral administration comprising an anti-allergic effective amount of descarbonylethoxyloratadine in a pharmaceutically acceptable carrier medium comprising a DCL-protective amount of a pharmaceutically acceptable basic salt and an amount of at least one disintegrant sufficient to provide dissolution of at least about 80% by weight of the pharmaceutical composition in about 45 minutes.

This invention also provides a pharmaceutical composition for oral administration comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least about 80% by weight of the pharmaceutical composition in about 45 minutes.

In a preferred embodiment, this invention provides a pharmaceutical composition for oral administration comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least about 80% by weight of the pharmaceutical composition in about 45 minutes, and which contains less than about 1% by weight of N-formyldescarbonyl-ethoxyloratadine.

This invention further provides a referred pharmaceutical composition for oral administration comprising:

| Ingredient | Amount (weight %) |
|---|---|
| Descarbonylethoxyloratadine | about 0.5 - 15 |
| Calcium Dibasic Phosphate | |
| Dihydrate USP | about 10 - 90 |
| Microcrystalline Cellulose NF | about 5 - 60 |
| Corn starch NF | about 1- 60 |
| Talc USP | about 0.5 - 20 |

This invention also provides another preferred pharmaceutical composition for oral administration comprising:

| Ingredient | Amount (weight %) |
|---|---|
| Descarbonylethoxyloratadine | about 0.5 -15 |
| Calcium Dibasic Phosphate | |
| Dihydrate USP | about 45 - 60 |
| Microcrystalline Cellulose NF | about 20 - 40 |
| Corn starch NF | about 5 - 15 |
| Talc USP | about 1-10 |

This invention also provides another preferred pharmaceutical composition for oral administration comprising:

| Ingredient | Amount (weight %) |
|---|---|
| Descarbonylethoxyloratadine | about 1-10 |
| Calcium Dibasic Phosphate Dihydrate USP | about 50 - 56 |
| Microcrystalline Cellulose NF | about 25 - 35 |
| Corn Starch NF | about 10 - 12 |
| Talc USP | about 2 - 5 |

The pharmaceutical compositions of the present invention are useful for treating allergic reactions in mammals.

### Detailed Description of the Invention

During the development of the compositions of the present invention, descarbonylethoxyloratadine was found to discolor when stored at 75% relative humidity ("RH") and a temperature of 40°C, alone or in combination with various excipients, such as those disclosed in U.S. Patent Nos. 4,657,716 and 5,595,997. We discovered that this color instability in the active ingredient was apparently due to a very minute amount of a degradation product caused by the presence of a wide variety of excipients commonly used in oral formulations - especially a tablet formulation. These excipients found unsuitable include acidic excipients including, but not limited to, stearic acid, povidone, and crospovidone, and other acidic excipients having a pH in water fewer than 7, and preferably in the range of about 3 to 5 as well as other excipients such as lactose, lactose monohydrate, sodium benzoate, and Glyceryl Behenate NF sold under the tradename of Compritol 888 .The presence of acidic excipients such as stearic acid in a solid powder formulation blend (similar to that of Example 6) containing DCL, lactose monohydrate, and stearic acid resulted in a large amount (14%) of decomposition of descarbonylethoxyloratadine after one week at 40°C and 75 % RH. When the pharmaceutical compositions of the present invention were subjected to the same stressed conditions for a longer period of time, i.e., 3 months, less than about 1% decomposition of descarbonylethoxyloratadine was found in the pharmaceutical compositions of the present invention. See Examples 1-5, 6 and 10 hereinafter. Preferably, the pharmaceutically acceptable carrier medium used in the pharmaceutical compositions of the preset invention should be substantially free, i.e., contain less than about 1 % by weight, of acidic excipients.

The major decomposition product of DCL found in the pharmaceutical compositions of the present invention is N-formylDCL. The pharmaceutical compositions of the present invention contain less than about 1% by weight, initially and at periods up to at least 24 months. Preferably, the pharmaceutical compositions of the present invention contain less than about 0.8 % by weight, and more preferably they less than about 0.6 % by weight of N-formylDCL when such compositions were stored at about 25°C and about 60 % RH for at least 24 months.

The term"pharmaceutically acceptable basic salts" as used herein means a calcium, magnesium or aluminum salt, or mixtures thereof, including, but not limited to carbonates, phosphates, silicates and sulfates of calcium, magnesium and aluminum. Typically suitable pharmaceutically acceptable basic salts include calcium sulfate anhydrous, hydrates of calcium sulfate, such as calcium sulfate dihydrate, magnesium sulfate anhydrous, hydrates of magnesium sulfate, dibasic calcium phosphate, dibasic calcium phosphate anyhdrous, tribasic calcium phosphate, calcium silicate, magnesium silicate, magnesium trisilicate, aluminum silicate, and magnesium aluminum silicate. The use of calcium phosphate salts is preferred. The use of Dibasic calcium phosphate hydrates is more preferred The use of dibasic calcium phosphate dihydrate is most preferred.

The DCL-protective amount of the pharmaceutically acceptable basic salt used in the compositions of the present invention is normally about 50% by weight of the total composition. The w/w ratio of the protective amount of the pharmaceutically acceptable basic salt to the anti-allergic amount of DCL is in the range of about 5:1 to about 60:1, preferably about 7:1 to about 11:1, and most preferably about 10:1 to about 11:1.

The term "disintegrant" as used herein means a pharmaceutically acceptable material or combination of such materials that provides a pharmaceutically acceptable dissolution rate for the compositions of the present invention, preferably a dissolution rate for the compositions of the present invention of at least about 80% by weight in about 45 minutes in accordance with the USP paddle dissolution test <711> on page s 1791-1793 of USP 23/ NF 18,1995, UNITED STATES PHARMA-COPEIAL CONVENTION, INC., Rockvile MD 20852. Normally, the dissolution rate is measured in 0.1N HCl at 37°C. The preferred dissolution rate of the compositions of the present invention is at least about 80 % by weight in about 30 minutes, and more preferably, the dissolution rate of the compositions of the present invention is at least about 90% by weight in about 30 minute.

Typically suitable pharmaceutically acceptable disintegrants include microcrystalline cellulose, starch, e.g., pregelatinized starch and corn starch, mannitol, croscarmellose sodium and confectioner's sugar (a mixture of at least 95% by weight sucrose and corn starch that has been ground to a fine powder).The pharmaceutical compositions of the present invention contain at least one, preferably at least two, and most preferably two pharmaceutically acceptable disintegrants in the w/w ratio of about 1:1 to 3:1. In a preferred embodiment of the present invention, the two pharmaceutically acceptable disintegrants are cellulose, and starch, preferably corn starch, in the w/w ratio of about 2:1 to about 3:1.

The w/w ratio of the protective amount or the pharmaceutically acceptable basic salt to the amount of the pharmaceutically acceptable disintegrant(s) is in the range of about 1.1:1 to about 2:1, preferably about 1.2:1 to about 1.75:1, and most preferably about 1.20:1 to about 1.25:1.

Unexpectedly, we discovered that when descarbonyl-ethoxyloratadine was combined with a carrier medium comprising dibasic calcium phosphate, and microcrystalline cellulose-in the absence of prior art excipients such as stearic acid, or lactose - we produced a pharmaceutical composition that was stable to discoloration when stored for 4 weeks in open petri dishes at a temperature of 40°C and relative humidity of 75%. In a preferred embodiment of the present invention, the carrier medium also contains corn starch and talc. In place of the corn starch one may substitute pregelatinized starch; the talc may be replaced by PEG 8000. The calcium dibasic phosphate may be replaced by calcium sulfate dihydrate, but use of calcium dibasic phosphate is preferred. No significant changes (less than about 1-2% by weight) were observed in the physical appearance, moisture content, chemical assay of descarbonylethoxyloratadine and dissolution rate of the tablet formulations when a preferred embodiment of the present invention of Example 10 was stored in plastic bottles or blister packages for up to 9 months at 25°C/60% RH or at 30°C/60 % RH or for up to 6 months at 40°C/75 % RH.

The descarbonylethoxyloratadine used in the present invention may be prepared in accordance with Example VI of U.S. Patent No. 4,659,716. Descarbonylethoxyloratadine exists in two polymorphic forms (form 1 and form 2) which may be prepared in accordance with the Examples 1-3 and procedures of commonly-owned co-pending U.S. Patent Application Serial No. 08/886,766 filed 07/02/97. These two polymorphic forms interconverted during the manufacture of the tablets formulation of the present invention. While either polymorph form may be used, form I is preferred.

### Pharmaceutical Compositions

Pharmaceutical compositions of this invention contain an anti-allergically effective amount of descarbonylethoxyloratadine as the active ingredient, and a pharmaceutically acceptable carrier medium which may include, in addiction to specific amounts of calcium dibasic phosphate and microcrystalline cellulose, other inert pharmaceutically acceptable ingredients that may be solids or liquids. Solid form compositions include powders, tablets, dispersible granules, capsules, cachets, and suppositories. The inert pharmaceutically acceptable carrier medium includes one or more substances which may also act as diluents, flavoring agents, solubilizers, lubricants, suspending agents, binders, tablet disintegration agents or encapsulating materials. The solid dosage forms of the pharmaceutical compositions of the present invention are suitable for oral administration and include powders, tablets, dispersible granules, capsules, cachets, buccals, and suppositories. In powders, the carrier medium is a finely divided solid which is in admixture with the finely divided active ingredient. In the tablet, the active ingredient is mixed with carrier medium having the necessary binding properties in suitable proportions and compacted in the shape and size desired. The anti-allergic effective amount of DCL in the pharmaceutical compositions of this invention, e.g., powders and tablets is from about 0.5 to about 15 percent , preferably about 0.5 to 10 weight percent, and more preferably about 1 to 10 weight percent. The term "compositions" is intended to include the formulation of the active compound with encapsulating material as carrier providing a capsule in which the active ingredient (with or without other carriers) is surrounded by carrier medium, which is thus in association with it. Similarly, caches are included.

The anti-allergic effective amount of descarbonylethoxyloratadine for oral administration varies from about 1 to 50 mg/day, preferably about 2.5 to 20 mg/ day and more preferably about 5 to 10 mg/day in single or divided doses. The most preferred amount is 5 mg, once a day.

Of course the precise dosage and dosage regimen may be varied depending upon the requirements of the patients (e.g.. his or her sex, age) as well as the severity of the allergic condition being treated. Determination of the proper dosage and dosage regimen for a particular patient will be within the skill of the attending clinician.

Descarbonylethoxyloratadine possess antihistaminic properties. These antihistaminic properties have been demonstrated in standard animal models, such as prevention of histamine - induced lethality in guinea pigs, Antihistaminic activity of polymorph form 1 and form 2 of descarbonyl-ethoxyloratadine has also been demonstrated in a monkey model.

### General Experimental

Descarbonylethoxyloratadine may be prepared in accordance with Example VI of USP No. 4,659,716. Calcium dibasic phosphate dihydrate [Ca(H₂PO₄)₂•2H₂O] is available from Rhone Poulenc Rorer, Shelton, CT 06484; microcrystalline cellulose is available from FMC Corporation Food & Pharmaceutical Products, Philadelphia, PA 19103, corn starch NF is available from National Starch & Chemical Corp., Bridgewater, NJ 08807 and the talc USP is available from Whittaker, Clark and Daniels, Inc., South Plainfield, NJ 07080.

### Method of Manufacture of Pharmaceutical Compositions of the Present Invention in the form of Tablets

The following procedure illustrates the formulation of tablets:

### Starch paste preparation

1. Prepare a 10 w/w starch paste by dispersing the paste portion of corn starch into a portion of purified water in a suitable container equipped with an agitator.
2. While mixing, heat the contents of the container to 95°C and maintain this temperature for 30 minute.
3. Add an additional amount of purified water to the heated mixture and allow the so-formed starch paste to cool to approximately 50°C.
4. While mixing, add the descarbonylethoxyloratadine to the starch paste.
   Granulation
5. To a suitable fluid bed processing bowl, charge the dibasic calcium phosphate dihydrate, a portion of the corn starch and a portion of the microcrystalline cellulose. Place the processing bowl into a fluid bed processor.
6. Fluidize the powder bed and mix for 3 minutes.
7. Begin granulating the powder by pumping the starch paste of step 4 into the fluidized bed at a suitable spray rate (for a 600,000 tablet batch size, the: spray rate was 500 mL/min.) and a bed temperature of 22°C.
8. Continue to dry the granulation at 60°C until the granulation has a final loss on drying of 2% or less.
9. Pass the dried granulation through a suitable sieve or mill.
10. Charge the granulation to a suitable blender and add the requisite amount of the remaining portion of microcrystalline cellulose, corn starch, and talc. Blend for 5 minutes to produce a uniform powder blend.

The resulting blend may be filled into suitable two-piece hard gelatin capsules on a suitable encapsulating machine. The blend may also be compressed to an appropriate size and weight on a suitable tablet machine.

### Tableting

1. Compress the final powder blend on a suitable tablet press with a target tablet weight of 100 mg and hardness of 7-9 s.c.u. (Strong-Cobb Units)

The tablets may be film-coated by charging the compressed tablets into suitable coating equipment having a rotating pan and heater. The tablets on the rotating pan are contacted at a temperature of about 30-50°C with a coating solutions formed by dissolving dear or colored coating materials in purified water. After the tablets are completely coated, a polishing powder may be added to the coated tablets to provide polished coated tablets. Alternatively, 'the colored coating material may be added as a dry powder in step 5 or 10, preferably step 5 of the Granulation phase of the process. It is preferred that the colored coating material is preferably substantially free, i.e., < about 1%, or more preferably completely free of offensive excipients such as lactose.

### Examples 1-5

Follow the above-listed manufacturing procedure using the ingredients listed below and compress the powder blend into tablets.

| Ingredients | 1mg strength | 2.5 mg strength | 5mg strength | 75 mg strength | 10mg strength |
|---|---|---|---|---|---|
| | (mg/tab) | (mg/tab) | (mg/tab) | (mg/tab) | (mg/tab) |
| Descarbonyl-ethoxyloratadine | 1 | 2.5 | 5 | 7.5 | 10 |
| | | | | | |
| Dibasic calcium phosphate dihydrate USP | 53 | 53 | 53 | 53 | 53 |
| | | | | | |
| Microcrystalline cellullose NF | 32 | 30.5 | 28 | 25.5 | 23 |
| | | | | | |
| Corn starch NF | 11 | 11 | 11 | 11 | 11 |
| | | | | | |
| Talc USP | 3 | 3 | 3 | 3 | 3 |
| | | | | | |
| Total | 100 | 100 | 100 | 100 | 100 |

### EXAMPLES 6-9

The tablet formulations of Examples 6-9 were prepared in accordance with procedure of Examples 1-5.

### Example 6

| Ingredients | mg/tablet |
|---|---|
| Descarbonylethoxyloratadine | 10 |
| Lactose monohydrate | 69 |
| Corn starch | 18 |
| Stearic acid | 2 |
| Silicon dioxide | 1 |

### Example 7

| Ingredients | mg/tablet |
|---|---|
| Descarbonylethoxyloratadine | 10 |
| Lactose monohydrate | 59 |
| Microcrystalline cellulose | 8 |
| Pregelatinized starch | 15 |
| Croscarmellose sodium | 5 |
| Silicon dioxide | 1 |
| Stearic acid | 2 |

### Example 8

| Ingredients | mg/tablet |
|---|---|
| Descarbonylethoxyloratadine | 25 |
| Dibasic calcium phosphate | 78.5 |
| Dihydrate | |
| Corn starch | 18 |
| Magnesium stearate | 1 |

### Example 9

| Ingredients | mg/tablet |
|---|---|
| Descarbonylethozyloratadine | 2.5 |
| Microcrystalline cellulose | 10 |
| Mannitol | 71.5 |
| Pregelatinized starch | 15 |
| Magnesium stearate | 1 |

The tablet formulations of Examples 6-9 prepared in accordance with procedure of Examples 1-5 discolored rapidly when they were placed in open petri dishes after less than one week under a temperature of 40°C and a relative humidity of 75%.

### Color stability of formulated tablets of Examples 1-5

The color stability of the above mentioned tablets of Examples 1-5 was studied in open petri dishes under a stressed condition of a temperature of 40°C and 75% relative humidity. After storage in the open petri dishes under this condition for 4 weeks, the tablets of Examples 1-5 were found to be free of discoloration and remained white in color. When descarbonyl-ethoxyloratadine was formulated with other excipients such as lactose and stearic acid and formed into tablets, in accordance with the procedures of Examples 1-5, the tablets of Examples 6-9 discolored rapidly after less than one week under the same storage conditions. A solid powder formulation blend(similar to that of the tablets of Examples 6) containing DCL, lactose monohydrate and stearic acid in the w/w/w/ ratio of 1:7:0.2. also decomposed rapidly after less than one week under the same storage conditions of a temperature of 40°C and 75% relative humidity; the chemical assay for descarbonylethoxyloratadine in this solid powder formulation was about 86% of the initial amount and the color of the formulation was pink.

### Example 10

The procedures of Examples 1-5 were followed except that the formulation of Example 3 was compressed into tablets and filmed coated and polished.

| Ingredients | mg/tablet |
|---|---|
| Descarbonylethoxyloratadine | 5.0 |
| Dibasic calcium phosphate | 53.00 |
| Dihydrate USP | |
| Microcrystalline cellulose NF | 28.00 |
| Corn starch NF | 11.00 |
| Talc NF | 3.00 |
| Film coat(blue) | 6.00 |
| Film coat(clear) | 0.6 |
| Polishing Wax¹ | 0.01 |

| | |
|---|---|
| 1. The polishing wax is a 1:1 w/w mixture of Camuba wax and white wax. | |

### The stability of formulated tablets of Example 10

Chemical assay, physical properties, and photostability of the formulated tablets of Examples 10 were measured on samples placed in high density polyethylene bottles and blister packages.

No significant changes (<1-2%) were observed in the physical appearance, moisture content, chemical assay of descarbonylethoxyloratadine and dissolution rate when the tablets of Example 10 was stored in plastic bottles or blister packages for up to 9 months at 25°C/60% relative humidity("RH") or at 30°C/60 % RH or for up to 6 months at 40°C/75 % RH. A small amount of degradation, e.g., N-formylDCL, was observed in the tablets stored in bottles (about 0.8%) and in blisters( about 1.2%) at 40°C/75 % RH for 6 months; only about 0.2-0.3% of the degradation product was observed in any samples of the tablets stored in blisters or bottles for 9 months at 25°C%60% or at 30°C/60% RH. It is expected that the International Conference on Harmonization("ICH") Impurity Guideline for a 5 mg tablet stored for 24 months at 25°C/60% RH or for 12 months at 30°C/60 % RH of 1% by weight of the tablet will be met. When the tablets in an open dish were subjected to ICH light conditions for one week at 25°C, the total amount of decomposition products was 0.34% by weight.

### Example 11

The procedures of Examples 10 were followed except that the Blue lake was added as a dry powder to step 5 of the Granulation phase and the formulation was thereafter compressed into tablets

| Ingredients | mg/tablet |
|---|---|
| Descarbonylethoxyloratadine | 5.0 |
| Dibasic calcium phosphate | 53.00 |
| Dihydrate USP | |
| Microcrystalline cellulose NF | 27.72 |
| Corn starch NF | 11:00 |
| Talc NF | 3.00 |
| FD&C Blue #2 Lake | 0.28 |
| Total | 100.00 |

The formulation of Example 11 is expected to have similar chemical assay, and physical properties and photostability to that observed for the formulation of Example 10.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

The present invention can be summarized by reference to the following embodiments (embs):
emb. 1. A pharmaceutical composition for oral administration comprising an anti-allergic effective amount of descarbonylethoxy-loratadine in a pharmaceutically acceptable carrier medium comprising a DCL-protective amount of a pharmaceutically acceptable basic salt and at least one pharmaceutically acceptable disintegrant.
emb. 2. The pharmaceutical composition of emb 1 wherein the at least one pharmaceutically acceptable disintegrant is in an amount sufficient to provide dissolution of at least 80% by weight of the pharmaceutical composition in about 45 minutes.
emb. 3. The pharmaceutical composition of emb. 1 wherein the w/w ratio of the DCL-protective amount of the pharmaceutically acceptable basic salt to said disintegrant is in the range of about 1:1 to 2:1, preferably about 1.5:1 to about 2:1, and preferably about 1.25:1 to about 1.75:1.
emb. 4. The pharmaceutical composition of emb. 1 wherein the pharmaceutically acceptable basic salt is a calcium, magnesium or aluminum salt, or mixtures thereof.
emb. 5. The pharmaceutical composition of emb. 1 wherein the pharmaceutically acceptable basic salt is a calcium phosphate salt.
emb. 6. The pharmaceutical composition of emb. 1 wherein the pharmaceutically acceptable carrier medium is substantially free of acidic excipients.
emb. 7. A pharmaceutical composition of emb. 1 which contains less than about 1% by weight of N-formyldescarbonylethoxyloratadine after storage at about 25°C and about 60% relative humidity for at least 24 months.
emb. 8. The pharmaceutical composition of emb. 1 wherein the w/w ratio of the DCL-protective amount of the pharmaceutically acceptable basic salt to the anti-allergic effective amount descarbonylethoxyloratadine is the range of about 5:1 to about 60:1, preferably about 7:1 to about 11:1, and most preferably about 10:1 to about 11:1.
emb. 9. A pharmaceutical composition for oral administration comprising an anti-allergic effective amount of descarbonylethoxy-loratadine in a pharmaceutically acceptable carrier medium comprising a DCL-protective amount of calcium dibasic phosphate, an amount of microcrystalline cellulose and of starch sufficient to provide dissolution of at least about 80% by weight of the pharmaceutical composition in about 45 minutes.
emb. 10. A pharmaceutical composition of emb. 9 which contains less than about 1% by weight of N-formyldescarbonylethoxyloratadine after storage at about 25°C and about 60% relative humidity for at least 24 months.
emb. 11. A pharmaceutical composition for oral administration comprising an anti-allergic effective amount of descarbonylethoxy-loratadine in a pharmaceutically acceptable carrier medium comprising a DCL-protective amount of calcium dibasic phosphate, an amount of microcrystalline cellulose and of starch sufficient to provide dissolution of at least 80% by weight of the pharmaceutical composition in about 45 minutes, and containing less than about 1% by weight of N-formyldescarbonyl-ethoxyloratadine after storage at about 25°C and about 60% relative humidity for at least 24 months.
emb. 12. A pharmaceutical composition of emb. 9 which comprises:

| Ingredients | Amount (weight %) |
|---|---|
| Descarbonylethoxyloratadine | about 0.5-15 |
| Calcium Dibasic Phosphate | |
| DihydrateUSP | about 10-90 |
| Microcrystalline Cellulose NF | about 5-60 |
| Corn starch NF | about 1-60 |
| Talc USP | about 0.5-20 |

emb. 13. A pharmaceutical composition of emb. 9 which comprises:

| Ingredients | Amount (weight %) |
|---|---|
| Descarbonylethoxyloratadine | about 0.5-15 |
| Calcium Dibasic Phosphate | |
| DihydrateUSP | about 45-60 |
| Microcrystalline Cellulose NF | about 20-40 |
| Corn starch NF | about 5-15 |
| Talc USP | about 1-10. |

emb. 14. A pharmaceutical composition of any preceding claim wherein the amount of descarbonylethoxyloratadine is in the range of about 1 to about 10 weight percent.
emb. 15. A pharmaceutical composition of emb. 9 which comprises:

| Ingredients | Amount (weight %) |
|---|---|
| Descarbonylethoxyloratadine | about 1-10 |
| Calcium Dibasic Phosphate Dihydrate USP | about 50 - 56 |
| Microcrystalline. Cellulose NF | about 25 - 35 |
| Corn Starch NF | about 10-12 |
| Talc USP | about 2-5 |

emb. 16. A pharmaceutical composition of any preceding claim which initially contains less than about 1% by weight of N-formyldescarbonylethoxyloratadine.
emb. 17. A pharmaceutical composition comprising an anti-allergic effective amount of descarbonylethoxyloratadine in a pharmaceutically acceptable carrier medium comprising a DCL-protective amount of a pharmaceutically acceptable basic salt.
emb. 18. The pharmaceutical composition of emb. 17 wherein said composition further comprises at least one pharmaceutically acceptable disintegrants.
emb. 19. The pharmaceutical composition of emb. 18 wherein the at least one pharmaceutically acceptable disintegrant is in an amount sufficient to provide dissolution of at least about 80% by weight of the pharmaceutical composition in about 45 minutes.
emb. 20. The pharmaceutical composition of emb. 19 wherein the w/w ratio of the DCL-protective amount of the pharmaceutically acceptable basic salt to said disintegrant is in the range of about 1:1 to 2:1, preferably about 1.5:1 to about 2:1, and preferably about 1.25:1 to about 1.75:1.
emb. 21. The pharmaceutical composition of emb. 18 wherein the pharmaceutically acceptable basic salt is a calcium, magnesium or aluminum salt, or mixtures thereof.
emb. 22. The pharmaceutical composition of emb. 18 wherein the pharmaceutically acceptable basic salt is a calcium phosphate salt.
emb. 23. The pharmaceutical composition of emb. 18 wherein the pharmaceutically acceptable carrier medium is substantially free of acidic excipients.
emb. 24. A pharmaceutical composition of emb. 18 which contains less than about 1% by weight of N-formyldescarbonylethoxyloratadine.
emb. 25. The pharmaceutical composition of emb. 18 wherein the w/w ratio of the DCL-protective amount of the pharmaceutically acceptable basic salt to the anti-allergic effective amount of descarbonylethoxyloratadine is the range of about 5:1 to about 60:1, preferably about 7:1 to about 11:1, and most preferably about 10:1 to about 11:1.
emb. 26. A pharmaceutical composition comprising an anti-allergic effective amount of descarbonylethoxyloratadine in a pharmaceutically acceptable carrier medium wherein said composition contains less than about 1% by weight of N-formylDCL, preferably less than about 0.8% of N-formylDCL, and more preferably less than about 0.6% of N-formyIDCL.
emb. 27. The pharmaceutical composition of emb. 26 wherein said composition is adapted for oral administration.
emb. 28. The pharmaceutical composition of emb. 26 wherein said composition has been stored at about 25°C and about 60% relative humidity for at least 24 months.

## Claims

1. A pharmaceutical composition for oral administration comprising an anti-allergic effective amount of descarbonylethoxy-loratadine in a pharmaceutically acceptable carrier medium comprising a DCL-protective amount of a pharmaceutically acceptable basic salt and at least one pharmaceutically acceptable disintegrant.

2. A pharmaceutical composition for oral administration comprising an anti-allergic effective amount of descarbonylethoxy-loratadine in a pharmaceutically acceptable carrier medium comprising a DCL-protective amount of calcium dibasic phosphate, an amount of microcrystalline cellulose and of starch sufficient to provide dissolution of at least about 80% by weight of the pharmaceutical composition in about 45 minutes.

3. A pharmaceutical composition of claim 1 or claim 2 which contains less than about 1% by weight of N-formyldescarbonyl-ethoxyloratadine after storage at about 25 °C and about 60% relative humidity for at least 24 months.

4. A pharmaceutical composition for oral administration comprising an anti-allergic effective amount of descarbonylethoxy-loratadine in a pharmaceutically acceptable carrier medium comprising a DCL-protective amount of calcium dibasic phosphate, an amount of microcrystalline cellulose and of starch sufficient to provide dissolution of at least 80% by weight of the pharmaceutical composition in about 45 minutes, and containing less than about 1% by weight of N-formyldescarbonyl-ethoxyloratadine after storage at about 25 °C and about 60% relative humidty for at least 24 months.

5. A pharmaceutical composition of any preceding claim wherein the amount of descarbonylethoxy-loratadine is in the range of about 1 to about 10 weight percent.

6. A pharmaceutical composition comprising an anti-allergic effective amount of descarbonylethoxy-loratadine in a pharmaceutically acceptable carrier medium comprising a DCL-protective amount of a pharmaceutically acceptable basic salt.

7. The pharmaceutical composition of claim 6, wherein said composition further comprises at least one pharmaceutically acceptable disintegrant.

8. The pharmaceutical composition of claim 1 or claim 7, wherein the at least one pharmaceutically acceptable disintegrant is in an amount sufficient to provide dissolution of at least about 80% by weight of the pharmaceutical composition in about 45 minutes.

9. The pharmaceutical composition of claim 1 or claim 8, wherein the w/w ratio of the DCL-protective amount of the pharmaceutically acceptable basic salt to said disintegrant is in the range of about 1:1 to 2:1, preferably about 1.5:1 to about 2:1, and preferably about 1.25:1 to about 1.75:1.

10. The pharmaceutical composition of claim 1 or claim 7, wherein the pharmaceutically acceptable basic salt is a calcium, magnesium or aluminum salt, or mixtures thereof.

11. The pharmaceutical composition of claim 1 or claim 7, wherein the pharmaceutically acceptable basic salt is a calcium phosphate salt.

12. The pharmaceutical composition of claim 1 or claim 7, wherein the pharmaceutically acceptable carrier medium is substantially free of acidic excipients.

13. A pharmaceutical composition of any preceding claim which contains less than about 1% by weight of N- formyldescarbonylethoxyloratadine.

14. The pharmaceutical composition of claim 1 or claim 7, wherein the w/w ratio of the DCL-protective amount of the pharmaceutically acceptable basic salt to the anti-allergic effective amount of the descarbonylethoxy-loratadine is the range of about 5:1 to about 60:1, preferably about 7:1 to about 11:1, and most preferably about 10:1 1 to about 11:1.

15. A pharmaceutical composition comprising an anti-allergic effective amount of descarbonylethoxy-loratadine in a pharmaceutically acceptable carrier medium wherein said composition contains less than about 1% by weight of N-formylDCL, preferably less than about 0.8% of N-formylDCL, and more preferably less than about 0.6% of N-formylDCL.
